# EUROPEAN PATENT APPLICATION

(11) **EP 4 667 942 A1**
(43) Date of publication of application: **24.12.2025**
(21) Application number: 24712877.0
(22) Date of filing: 13.02.2024
(51) Int. Cl.: G01N 33/72, G01N 21/00

(54) **IN VITRO METHOD FOR IDENTIFYING FREE RADICALS IN BLOOD AND THE USE THEREOF IN THE DIAGNOSIS OF PATHOLOGIES**

(30) Priority: 13.02.2023 ES 202330106
(71) Applicant: Universidad de Oviedo, 33003 Oviedo (ES)
(72) Inventor: QUIRÓS GONZÁLEZ, Isabel, 33003 Oviedo (ES); SÁINZ MENÉNDEZ, Rosa María, 33003 Oviedo (ES); MAYO BARRALLO, Juan Carlos, 33003 Oviedo (ES); HEVIA SÁNCHEZ, David, 33003 Oviedo (ES)
(74) Representative: Pons IP
(86) International application number: PCT/ES2024/070080
(87) International publication number: WO 2024/170811

(57) **Abstract**

The present invention describes a method for identifying free radicals present in a pathological process suffered by a subject comprising: (a) determining the absorption spectrum of haemoglobin (Hb) between 500 and 1000 nm in a sample of venous blood isolated from the subject, and (b) comparing the absorption spectrum obtained in step (a) with the absorption spectrum of Hb between 500 and 1000 nm obtained in a sample of venous blood from a control subject. This method is applicable in the diagnosis of pathologies such as endometriosis.

## Description

This invention relates to an in vitro method for detecting oxidation-reduction (redox) changes in mammalian blood. It also relates to a specific application in the diagnosis of diseases, such as endometriosis. The metho is based on the absorption spectrum of the haemoglobin in the near infrared (NIR). Therefore, the present invention belongs to the field of biology and biomedicine, with direct application in the diagnosis of diseases.

### BACKGROUND OF THE INVENTION

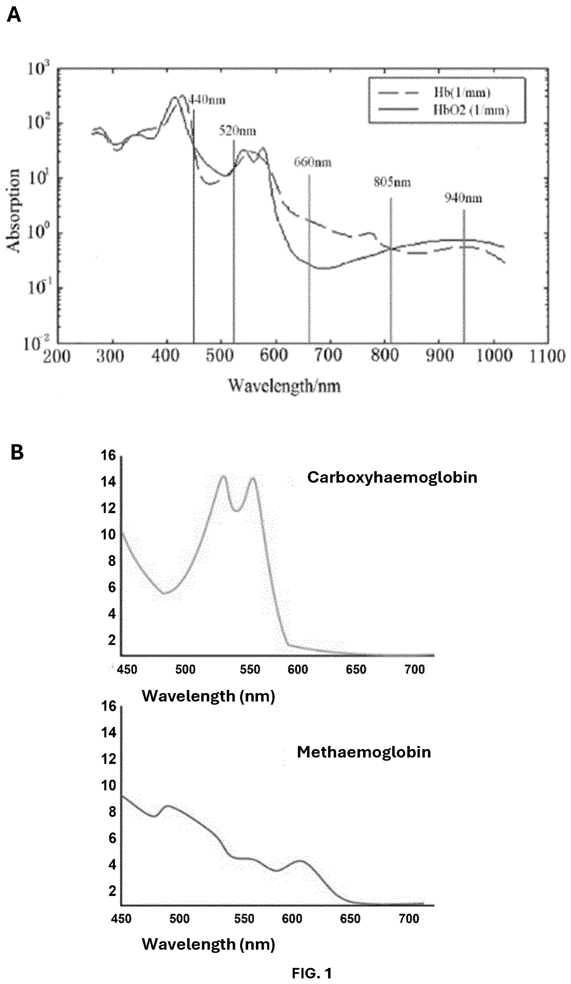

Haemoglobin is a protein with optical properties, it can absorb light from 250 to 1100 nm. The intensity of absorption depends on the concentration of the protein and its degree of oxygenation. It presents different absorption patterns that allows to distinguish between oxygenated and deoxygenated form, as well as chemical modifications as in the case of methaemoglobin and glycosylated haemoglobin based on the spectrum (see figure 1). These differences in the absorption pattern depend on their tertiary and quaternary structure. Haemoglobin is carried by the red blood cells (RBC) that live for 120 days. The RBCs are corpuscles (no nucleus) and are not currently assumed to be capable of protein biosynthesis once they reach their final stage of differentiation, so it is reasonable to assume that the haemoglobin they carry remains in the RBC from the beginning of their "life span" until the end.

Haemoglobin is a tetramer with an heme ring in the centre. Its amino-acid sequence has several positions sensitive to redox modifications. This patent application is based on the observation that specific free radicals affect specifically the absorption spectrum of haemoglobin in the NIR spectrum (500-1000nm). Free radicals are metabolites naturally occurring in aerobic living organisms. Apart from their production as a by-product of the electron respiratory chain, some cell types, such as lymphocytes, leukocytes or macrophages produce them for immune defence and wound healing response.

The free radical species O₂^{●-} , H₂O₂ and NO are the most common ones in non-pathological conditions and specially H₂O₂ and NO serve numerous cell and tissue regulation purposes. In pathological cellular models, it is known that these species are the first ones to be increased, initiating a "chain reaction" of redox reactions that can produce in turn advanced oxidation products that can cause sever damage to cells, tissues and organisms.

Considering all the above, the observation of the haemoglobin NIR spectrum is a reporter of the overall redox status of the individual in the 120 days prior to the measurement (which is the half-life of the erythrocyte).

There is currently no redox reporter validated for clinical use. Due to this lack of tools in the clinic, essential aspects of redox biology that have been demonstrated in vitro or in mouse models have never been confirmed in humans, where only advanced oxidation products can be monitored. There are no real-time markers of redox status that allow to distinguish different radicals in a single measurement, either for clinical or basic research (animal or in vitro). This would be critical to early stages of the disease where antioxidant intervention could be applied as a therapeutic strategy.

Endometriosis is a disabling gynaecological disorder that affects a variable percentage of the population. It is estimated that at least 5-15% of the female population suffers from this disease. The disease is caused by ectopic growth of the endometrium. The endometrium lies on top of the myometrium, which is a very thick and resistant muscular layer. When the proliferative phase occurs during the menstrual cycle, the endometrium gains five time more thickness, this growth is limited by the resistance of the myometrium. It should be noted that this proliferative phase is accompanied by an important angiogenic process (creation of new blood vessels). When the endometrial cysts settle ectopically in the ovary, fallopian tube, rectum, large intestine, vagina or mesentery, it perforates the underlying tissue causing pain, haemorrhage and inflammation among other symptoms.

Ultrasound imaging techniques are often used in the diagnosis of endometriosis, but it does not detect all the cases and requires from specific equipment and personnel. Blood tests are not routinely used for diagnosis, there are some markers such as CA-125 that are increased in a subset of women with endometriosis, but it shows some lack of sensitivity. Laparoscopy is an operative technique that allows observation of the abdominal cavity. In young patients, especially if the objective is fertility, the aim is to eliminate the disease while preserving as much healthy tissue as possible. In patients aged 45-50 and older the proposal is to remove damaged tissue and if indicated hysterectomy. In order to avoid laparoscopy when the ultrasound and manual examination fail to detect the cysts, alternative imaging techniques such as MRI or CT may be used.

Therefore, there is a need to provide new biomarkers for the diagnosis of redox related pathologies, such as endometriosis, that can be used in a diagnostic test that is rapid reliable and based on non-/low- invasive techniques that show high sensitivity and specificity.

### DESCRIPTION OF THE INVENTION

The inventors have observed that the absorption spectrum of haemoglobin in the NIR (500-1000 nm) varies in a specific manner when the haemoglobin present in an erythrocyte lysate is exposed to different free radicals. Thus, in the present invention, the absorption spectrum of haemoglobin in the NIR is presented as marker of the endogenous redox state of a subject and can provide information on the early free radicals involved in the process.

As the endogenous redox state is indicative of the pathology suffered by such subject, the absorption spectrum of haemoglobin can be used as a diagnostic marker for different pathologies in the clinic, as it will be demonstrated in the present invention in the case of endometriosis.

Thus, the present invention provides a new biomarker (NIR spectrum of haemoglobin), which allows the detection of free radicals, mainly early free radicals, at pathological level. It is the only existing method for direct and specific detection of early free radicals in vivo. Furthermore, it uses an endogenous "control", haemoglobin, and therefore it does not require any injection of contrast or probe. Because of this, there are no toxic or other side effects, and it does not require typical pharmacological tests (pharmacodynamics and distribution). Also, the use of haemoglobin NIR (500-1000nm) as pathology marker provides a simple and inexpensive form of diagnosis, which can be applied routinely, and it does not require specialised medical personnel. Overall, the present invention enables the rapid, reliable, and low-invasive diagnosis of pathologies suffered by a subject, such as endometriosis.

Therefore, in one aspect the present invention relates to an in vitro method for identifying free radicals present in a pathological process suffer by a subject, hereinafter "method of invention" comprising:
(a) Determining the absorption spectrum of haemoglobin (Hb) between 500 and 1000 nm in a sample of venous blood isolated from the subject, and
(b) Comparing the absorption spectrum obtained in step (a) with the absorption spectrum of Hb between 500 and 1000 nm obtained from a venous blood sample from a population not suffering from the pathological process,
   Wherein:
   - If the absorption spectrum of Hb determined in step (a) shows a significant decrease in absorbance at 955, 985, and 1000 nm, resulting in a decrease in the area under the curve between 955 and 1000 nm, then the major free radical is hydrogen peroxide (H₂O₂).
   - If the absorption spectrum of Hb determined in step (a) shows a significant increase in absorbance at 640, 655, 670, 685, 700 and 715 nm, resulting in an increase in the area under the curve between 640 and 715 nm, then the major free radical is peroxynitrite (-ONOO-),
   - If the absorption spectrum of Hb determined in step (a) shows a significant increase in absorbance at 625, 640, and 655 nm, resulting in an increase in the area under the curve between 625 and 655 nm, then the major free radical is superoxide (O₂^{●-})
   - If the absorption spectrum of Hb determined in step (a) shows a significant decrease over the spectrum between 600 and 1000, resulting in a decrease in the area under the curve between 600 and 1000 nm, then the major free radical is the nitric oxide (NO^{●-}).

The first stage of the method of the invention [stage (a)] comprises the determination of the absorption spectrum of haemoglobin between 500 and 1000 in a venous blood sample isolated from a subject.

The absorption spectrum is a graphical representation of the amount of light absorbed (ε) at different wavelengths (λ). The absorption spectrum can be measured by any of the techniques and instruments known in the state of the art. An example of instruments useful for the measurement of the absorption spectrum includes, but is not limited to, a spectrophotometer, such as the Varian CARY^{®}50 UV-Vis spectrophotometer. In the case of the present invention, the molecule whose absorption spectrum is measured is haemoglobin.

Haemoglobin is a tetrameric oxygen-carrying protein with a molecular mass of approximately 64,000 g/mol (64 KDa), form by four polypeptide chains (globins), each of them presents an heme group whose iron atom is capable of binding a dioxygen molecule in a reversible way.

First, in order to measure the absorption spectrum of haemoglobin, the haemoglobin has to be isolated from a sample of the subject's blood. In the present invention, "sample" means a part or small quantity of a thing which is considered to be representative of the whole. The sample is taken using certain methods to be subjected to study, analysis or experimentation. Thus, in the context of the present invention, the sample is a blood sample.

The blood sample can be obtained by venipuncture, e.g. routine collection of 5mL in EDTA tubes, or by fingerstick (puncture of the fingertip). Procedures and instrumentation for blood sampling or extraction are widely known to the skilled person in the art. In a particular embodiment of the method of the invention, the absorption spectrum of haemoglobin is measured on a blood sample from a routine collection or on a drop of blood and immediately lysed in distilled water. In case that the blood comes from a routine blood collection, the sample is centrifuged and after the removal of the serum volume, an equivalent volume of distilled water is added to obtain an homogeneous suspension. As the skilled person in the art knows, a sample treated in said way can be stored at -80°C until use.

Once the blood sample has been isolated, haemoglobin is isolated. Protein isolation techniques, in particular techniques to isolate haemoglobin from a blood sample, are widely known in the state of the art and are routine practice for the skilled person. Alternatively, however, the absorption spectrum of haemoglobin can be measured directly in the blood sample isolated from the subject.

As used herein, the term "subject" refers to all animals classified as mammals and includes, but is not limited to, domestic and farm animals, human primates, e.g. humans, non-human primates, cows, horses, pigs, sheep, goats, dogs, cats or rodents. Preferably, the subject from which the blood sample is to be taken to implement the method of the invention is a mammal, more preferably a primate or a canid, still more preferably a human being of any age.

Optionally, the method of the invention may comprise, prior to step (a), a step of processing the blood sample taken from the subject. Techniques and procedures for processing blood samples are common knowledge for the skilled person in the art. For example, in the case of starting from a blood sample stored at -80°C, 3 freeze-thaw cycles are required, and a subsequent centrifugation of, for example, 10 minutes at 10,000 g, discarding the erythrocyte membranes. For spectral and haemoglobin concentration measurements, a dilution of the supernatant can be performed, e.g. a 1:100 dilution. As the skilled person understands, in the case of starting from a drop of blood as a sample, sample processing is not necessary.

Once the blood sample or, alternatively, haemoglobin has been extracted from the blood sample, and the absorption spectrum of haemoglobin as defined in step (a) has been measured, the absorption spectrum obtained in step (a) is compared with the absorption spectrum of Hb between 500 and 1000 nm obtained in a venous blood sample from a population not suffering from the pathological process.

In the present invention, "population not suffering from the pathological process" means the set of subjects who do not present the pathological process whose free radicals are being identified. The haemoglobin absorption spectrum obtained from this population will be the average of the individual absorption spectra of each subject in the population.

Thus, it can be concluded from such comparison that:
- If the absorption spectrum of Hb determined in step (a) shows a significant decrease in absorbance at 955, 985, and 1000 nm, resulting in a decrease in the area under the curve between 955 and 1000 nm, then the major free radical is hydrogen peroxide (H₂O₂),.
- If the absorption spectrum of Hb determined in step (a) shows a significant increase in absorbance at 640, 655, 670, 685, 700 and 715 nm, resulting in an increase in the area under the curve between 640 and 715 nm, then the major free radical is peroxynitrite (-ONOO-),
- If the absorption spectrum of Hb determined in step (a) shows a significant increase in absorbance at 625, 640, and 655 nm, resulting in an increase in the area under the curve between 625 and 655 nm, then the major free radical is superoxide (O₂^{●}),⁻
- If the absorption spectrum of Hb determined in step (a) shows a significant decrease over the spectrum between 600 and 1000, resulting in a decrease in the area under the curve between 600 and 1000 nm, then the major free radical is the nitric oxide (NO^{●-}).

In the present invention, the terms "significant decrease" or "significant increase" refer to statistically significant decreases or increases. A person skilled in the art knows how to interpret unambiguously whether the change in a given value is statistically significant or not.

Therefore, by applying the method of the invention, the spectrum/pattern of free radicals present in a given pathology can be identified, being useful in the diagnosis of such pathology. Examples of pathologies include, but are not limited to, all those pathologies that involve an inflammatory process, and that involve a significant vascularisation of the tissue or occur in a highly vascularised tissue, such as endometriosis, cancer, inflammatory lung diseases (e.g., and not limited to, pneumonia), inflammatory diseases of the gastrointestinal tract (e.g., and not limited to, ulcerative colitis and Crohn's disease), inflammatory diseases of the kidney (e.g., and not limited to, glomerulo nephritis), inflammatory diseases of the skin (e.g., and not limited to, dermatitis). Nevertheless, in a particular embodiment of the method of the invention, alone or in combination with the rest of particular embodiments, the pathology is endometriosis, cancer, lung inflammatory disease, kidney inflammatory disease or skin inflammatory disease.

Considering all the above, in another aspect, the present invention relates to the *in vitro* use of the absorption spectrum of haemoglobin between 500 and 1,000 nm, hereinafter "use of the invention", for the identification of free radicals present in a pathological process suffered by a subject.

How to use the absorption spectrum of haemoglobin for the identification of free radicals present in a pathological process has been described above.

In a particular embodiment of the use of invention, the absorption spectrum of haemoglobin between 500 and 1,000 nm in the presence of free radicals comprises the following changes (compared to the spectrum of haemoglobin in the absence of free radicals):
- If the absorption spectrum of Hb shows a significant decrease in absorbance at 955, 985, and 1000 nm, resulting in a decrease in the area under the curve between 955 and 1000 nm, then the major free radical is hydrogen peroxide (H₂O₂),.
- If the absorption spectrum of Hb shows a significant increase in absorbance at 640, 655, 670, 685, 700 and 715 nm, resulting in an increase in the area under the curve between 640 and 715 nm, then the major free radical is peroxynitrite (-ONOO-),
- If the absorption spectrum of Hb shows a significant increase in absorbance at 625, 640, and 655 nm, resulting in an increase in the area under the curve between 625 and 655 nm, then the major free radical is superoxide ( O₂^{●}),**⁻**
- If the absorption spectrum of Hb shows a significant decrease over the spectrum between 600 and 1000 nm, resulting in a decrease in the area under the curve between 600 and 1000 nm, then the major free radical is the nitric oxide (NO^{●-}).

In a particular embodiment of the use of the invention, the subject is a mammal, in particular a primate or a canid, more particularly a human being.

In another particular embodiment of the use of the invention, the pathological process suffered by the subject is a disease involving inflammation. Examples of diseases involving inflammation have been mentioned in previously. In a more particular embodiment of the use of the invention, the pathological process is endometriosis.

As explained above, the identification of free radicals present in a given pathology can be used in the diagnosis of that pathology.

Therefore, in another aspect the present invention also encompasses an *in vitro* method for diagnosing the pathology suffered by a subject, hereinafter "diagnostic method of the invention", comprising:
(a) determining the spectrum/pattern of free radicals present in a venous blood sample isolated from a subject suffering from the pathology to be diagnosed by the method of the invention,
(b) determining the pattern of free radicals present in a venous blood sample isolated from a subject to be diagnosed by the method of the invention, and
(c) comparing the pattern of free radicals obtained in step (a) with the pattern of free radicals present in the subject to be diagnosed obtained in step (b),
wherein if the subject to be diagnosed has the same pattern of free radicals as the subject with the pathology, then the subject is diagnosed with the pathology.

As used in the present description, the term "diagnose" or "diagnosis" refers to the identification of the nature of an condition, disease or other problem by examination of symptoms, parameters or biomarkers in a subject. In the context of the present invention, the parameter used for diagnosis is the absorption spectrum of haemoglobin between 500 and 1000 nm which is indicative of the major free radicals present in the subject.

In a first stage of the diagnostic method of the invention [stage (a)], the method of invention is used to identify the free radicals present in a venous blood sample isolated from a subject suffering from the pathology to be diagnosed by the method of the invention. In the present invention, "free radical pattern" or "free radical spectrum" means the set of early free radicals present in a subject that characterise its oxidative state. If the subject suffers from a disease, preferably an inflammatory disease, this free radical pattern will be characteristic of the disease suffered.

Next, in step (b) of the diagnostic method of the invention, the free radicals present in a venous blood sample isolated from a subject to be diagnosed is determined by the diagnostic method of the invention. This subject will present a free radical pattern indicative of their oxidative state.

Therefore, by comparing the patterns in both subjects (that from the subject diagnosed with the disease and that from the subject to be diagnosed) it is possible to know whether the subject has the disease, because if the pattern in the subject to be diagnosed matches that of the subject with the disease, then the diagnosed subject has the disease.

Thus, the diagnostic method of the invention comprises a third stage (c) comprising comparing the pattern of free radicals obtained in stage (a) with the pattern of free radicals present in the subject to be diagnosed obtained in stage (b), where if the subject to be diagnosed has the same pattern of free radicals as the subject with the pathology, then the subject is diagnosed with the pathology.

How to implement the diagnostic method of the invention has been explained above, and such explanations, as well as the definitions of the terms employed and their particular embodiment, are applicable to the diagnostic method of the invention.

By using the diagnostic method of the invention, any pathology involving inflammation can be diagnosed. Examples of pathologies involving inflammation have been described in previous paragraphs. However, in a particular embodiment of the diagnostic method of the invention, the pathology to be diagnosed is endometriosis, cancer, an inflammatory lung disease, an inflammatory kidney disease, or an inflammatory skin disease. Examples of inflammatory diseases of the lung, kidney or skin have been described in previous paragraphs and are applicable to the present inventive aspect.

In the present invention, "endometriosis" means a reproductive disorder in women characterised by the presence of endometrial tissue outside its normal location in the uterus which displays a severe degree inflammation and thus production of free radicals, coming also from blood clots in haemorrhagic locations.

In a particular embodiment of the diagnostic method of the invention, if the pathology is endometriosis, then:
- the area under the absorbance curve between 625 and 655 nm for haemoglobin in the NIR is greater than the area under the curve obtained from a non-endometriosis subject, which is indicative of increased O2-- , and
- the area under the absorbance curve between 900 and 1000 nm for haemoglobin in the NIR is smaller than the area under the curve obtained from a non-endometriosis subject, which is indicative of a decrease in NO●.

Analogous to the diagnostic method of the invention, in another aspect the present invention also relates to the use of the absorption spectrum of haemoglobin between 500 and 1,000 nm for the *in vitro* diagnosis of a pathology in a subject, hereinafter "diagnostic use of the invention".

In a particular embodiment of the diagnostic use of the invention, the absorption spectrum of haemoglobin between 500 and 1,000 nm in the presence of free radicals comprises the following changes (compared to the spectrum of haemoglobin in the absence of free radicals):
- If the absorption spectrum of Hb shows a significant decrease in absorbance at 955, 985, and 1000 nm, resulting in a decrease in the area under the curve between 955 and 1000 nm, then the major free radical is hydrogen peroxide (H₂O₂),.
- If the absorption spectrum of Hb shows a significant increase in absorbance at 640, 655, 670, 685, 700 and 715 nm, resulting in an increase in the area under the curve between 640 and 715 nm, then the major free radical is peroxynitrite (-ONOO-),
- If the absorption spectrum of Hb shows a significant increase in absorbance at 625, 640, and 655 nm, resulting in an increase in the area under the curve between 625 and 655 nm, then the major free radical is superoxide (O₂^{●}),
- If the absorption spectrum of Hb shows a significant decrease over the spectrum between 600 and 1000 nm, resulting in a decrease in the area under the curve between 600 and 1000 nm, then the major free radical is the nitric oxide (NO**^{●-}**)**.**

Thus, in a particular embodiment of the diagnostic use of the invention, the subject is a mammal, in particular a primate or a canid, more particularly a human being.

In another particular embodiment of the diagnostic use of the invention, alone or in combination with the previous particular embodiment, the pathology is endometriosis, cancer, an inflammatory lung disease, an inflammatory kidney disease, or an inflammatory skin disease.

In another particular embodiment of the diagnostic use of the invention, alone or in combination with any or all of the above particular uses, if the pathology is endometriosis, then:
- the area under the absorbance curve between 625 and 655 nm for haemoglobin in the NIR is greater than the area under the curve obtained from a non-endometriosis subject, which is indicative of increased O₂^{●**-**}, and
the area under the absorbance curve between 900 and 1000 nm for haemoglobin in the NIR is smaller than the area under the curve obtained from a non-endometriosis subject, which is indicative of a decrease in NO.

### DESCRIPTION OF THE FIGURES

**Figure 1****.** NIR absorption spectrum of haemoglobin. A) Absorption spectrum of deoxygenated (Hb) and oxygenated (HbO2) haemoglobin. B) Absorption spectrum of chemical modifications of haemoglobin (carboxyhaemoglobin and methemoglobin).
**Figure 2****.** Absorption spectrum of haemoglobin in the presence of H₂O₂. Absorbance intensity (Abs i.u.) of human blood lysates between 500 and 1000 nm (n=5), samples were exposed to 750 µM H₂O₂ for 10 minutes. Values expressed as mean ± standard error.
**Figure 3****.** Absorption spectrum of haemoglobin in the presence of -ONOO-. Absorbance intensity (Abs i.u.) of human blood lysates between 500 and 1000 nm (n=3), samples were exposed to 30 µM -ONOO- for 10 minutes. Values expressed as mean ± standard error.
**Figure 4****.** Absorption spectrum of haemoglobin in the presence of O₂^{●-}. Absorbance intensity (Abs i.u.) of human blood lysates between 500 and 1000 nm (n=3), samples were exposed to 5mU XAN+500 mU/ml XO for 10 minutes. Values expressed as mean ± standard error.
**Figure 5****.** Absorption spectrum of haemoglobin in the presence of NO. Absorbance intensity (Abs i.u.) of human blood lysates between 600 and 1000 nm (n=3) incubated at 30 s. Values expressed as mean.
**Figure 6****.** Absorbance spectrum of haemoglobin in women with endometriosis. A) Absorbance pattern along the NIR of blood from women without (Control, CON; n=14) and with endometriosis (ENDO; n=10). Values expressed as mean ± standard error. B) Area Under the Curve (AUC) values of the NIR spectrum, mean is represented. C) ROC curve of the AUC parameter for the cohort.
**Figure 7****.** Area under the absorbance standard curve of control (CON=5) and endometriosis (E-=22) women with mild (E-L), moderate (E-M) or severe (E-S) degree of the disease. A) Area Under the Curve (AUC) values of the NIR spectrum, the mean is represented. B) ROC curve of the AUC parameter for patients suffering mild degree of the disease. C) ROC curve of the AUC parameter for patients suffering severe degree of the disease.

### EXAMPLES

For better understanding, t The present invention will now be illustrated by means of a assay carried out by the inventors which show the effectiveness of the invention through two examples of preferred embodiments, described in detail, , which are to be understood as not limiting the scope of the invention.

### Example 1 - Change in the absorption spectrum of haemoglobin due to the presence of free radicals.

Haemoglobin is a protein highly concentrated in blood. It has optical properties, absorbing light in the spectral range from 300 to 1100 (see Figure 1). In addition, haemoglobin is highly sensitive to changes in redox state and very reactive to such chemical modifications that may even alter its binding capacity to oxygen.

### MATERIAL AND METHODS

### Section 1: Obtaining a haemoglobin sample.

Mammalian erythrocytes (human or mouse) were obtained from venous blood collection. The collection was performed by puncture of mid or radial venous blood and vacuum tube with EDTA as coagulant. The sample was kept at 4°C until processing. The samples were centrifuged at 5000 g 10' to deposit the RBCs. Plasma was removed with a micropipette and an equivalent of water was added. The red was resuspended until a homogeneous suspension was obtained. After 3 freeze-thaw cycles (-80°C 5'- 37°C 10') the sample was centrifuged at 10 000 g at 4°C for 10' to remove membrane debris.

### Section 2: Measurement of haemoglobin concentration in blood lysate.

For the analysis of venous blood collection samples, samples were diluted 1/100 in distilled water. In this case, haemoglobin concentration was determined by colorimetric assay (Sigma MAK115) according to the manufacturer's instructions.

### Section 3: Measurement of NIR absorption spectrum of haemoglobin in blood lysate.

A volume of 100 µL of a 1/100 solution in distilled water was loaded into 96-well plates in duplicate. A light absorption reading between 500 and 1000 nm was taken every 5 nm on a Varian CARY^{®} 50 UV-Vis spectrophotometer.

### Section 4: Incubation with radical oxygen and nitrogen species.

Haemoglobin samples were incubated for 10 min at room temperature in the presence of the following compounds: 750 µM hydrogen peroxide (H₂O₂); 30 µM -ONOO-; 5 mU xanthine + 500 mU xanthine oxidase for O₂^{●-} generation; 1.4 nU NOS, 2.5 mM cofactor and substrate solution (abcam 211083) for nitric oxide generation.

### Section 5: Cohort of endometriosis study samples

Samples were obtained from fertile women treated at the gynaecology clinic and the in vitro fertilisation unit of the Hospital Universitario Central de Asturias. Blood samples were collected from women with and without endometriosis in two different cohorts (First: CON=14; ENDO=10/ Second: CON=5 ENDO=22). Patients signed the corresponding informed consent form (CEI PA 263/18). Endometriosis had previously been diagnosed by gynaecological palpation, ultrasound or MRI. Patients diagnosed with endometriosis were either not on contraceptive treatment or in contraceptive treatment resistance stage. The samples were treated as described in section 1 and their absorption spectrum was measured and the concentration was obtained following the procedure described in sections 2 and 3.

### RESULTS

### Changes in the haemoglobin spectrum due to H₂O₂.

The spectrum of haemoglobin changes significantly when exposed to H₂O₂. The absorbance at wavelengths 955, 985 and 1000 nm is significantly lower than the same range in blood samples not exposed to peroxide (table 1).

**Table 1. Significantly altered wavelengths (wl) in the NIR haemoglobin spectrum after H₂O₂ exposure.**

| **wl** | **955** | | **985** | | **1000** | |
|---|---|---|---|---|---|---|
| CON | 0.251 | ±0.002 | 0.193 | ±0.004 | 0.168 | ±0.004 |
| H₂O₂ | 0.0233 | ±0.005 | 0.179 | ±0.005 | 0.1524 | ±0.004 |
| p-value | 0.011 | | 0.034 | | 0.038 | |

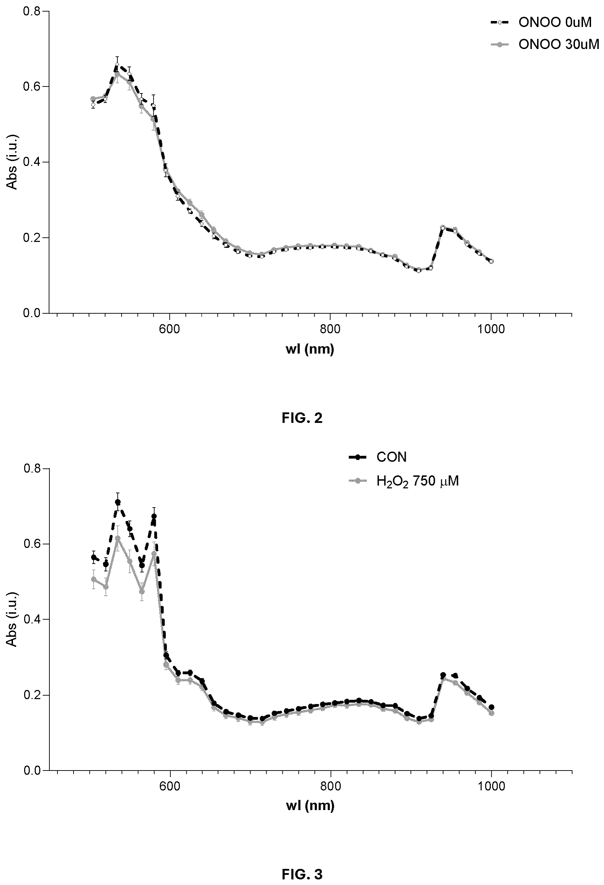

Since all other wavelengths in the range between 880 and 1000 are close to significance, it can be concluded that there is a general decrease in the height of the curve of the spectrum in that range. Thus, the area under the curve is significantly lower in the range between 880-1000 in the presence of H₂O₂, mean AUCCON= 28.81 C.I. [28.37-29.26]; mean AUCH2O2=32.14 C.I. [31.26-33.02] (Figure 2).

### Changes in the haemoglobin absorption spectrum due to -ONOO-.

To study the effect of -ONOO- on an erythrocyte lysate, changes were studied in the sample prior to the addition of -ONOO-, in the presence of the solvent (VEH). In the presence of - ONOO- haemoglobin varies significantly in a specific range as follows: between 640 and 715 (640, 655, 670, 685, 700 and 715), the absorbance increases in the presence of said radical (see Figure 3).

### Changes in the absorption spectrum of haemoglobin due to O₂^{●-}.

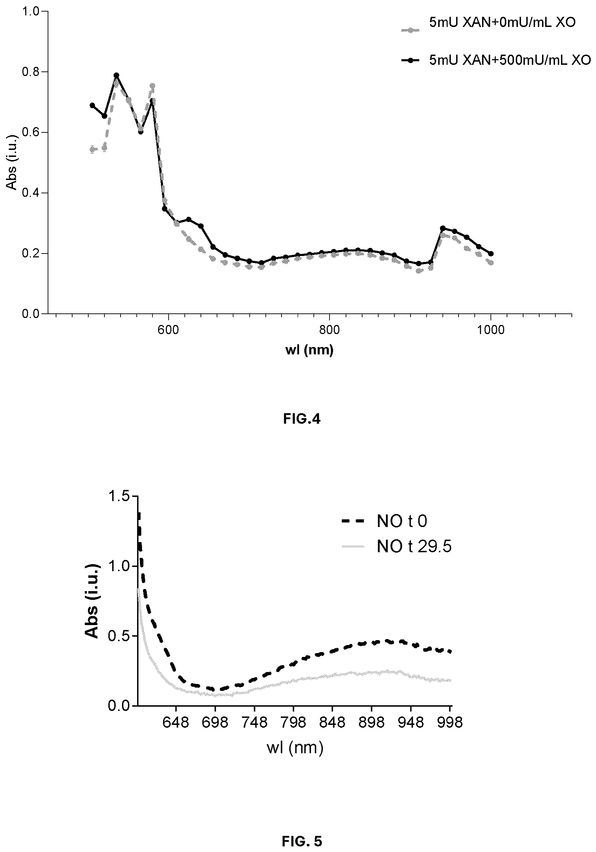

In the presence of O₂^{●-} erythrocyte lysate in distilled water varies the absorption spectrum of haemoglobin between 620 and 660 nanometres where its absorption spectrum will vary significatively and, specifically, in the wavelengths 625, 640 and 655 incrasing the absorbance in the presence of said radical. There is therefore an increase in the relative maximum observed between 625-655. Likewise, a significant change is also observed throughout the absorption range of wavelengths between 850-1000 with an increase in absorbance AUCXAN= 28.81 C.I. [28.37-29.26]; mean AUCXAN+XO=21.32 C.I. [31.26-33.02] (See Figure 4).

### Changes in the haemoglobin uptake spectrum due to nitric oxide.

In the presence of the nitric oxide radical, the absorption pattern drops absorbance intensity across the entire spectrum from 600 to 1000 nm (see Figure 5).

### CONCLUSION

Haemoglobin changes its absorption in the NIR spectrum when exposed to different types of oxygen-derived free radicals. Each type of radical alters the spectrum in specific areas; thus multispectral analysis of the absorbance of haemoglobin in the NIR spectrum is a tool to reconstruct the register of radicals to which it has been exposed.

### Example 2 - Application to the diagnosis of endometriosis.

From the results presented above, it can be stated that those pathological processes with a increase in free radicals and that have a vascular component, haemoglobin acquires the "signature" of the radicals involved in this pathological process, being capable of detecting the diseases from their initial stages. Furthermore, this signature would be specific for each pathology as different pathologies increase different groups of reactive species. As a proof of concept, we propose its application in the diagnosis of endometriosis.

### Haemoglobin absorbance pattern in patients with endometriosis

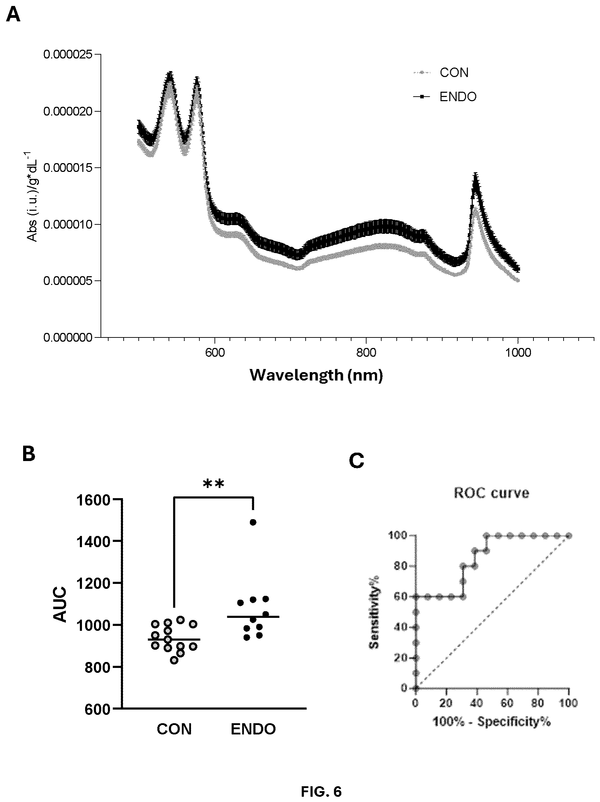

The NIR absorbance pattern of blood from women with endometriosis differs from that of women without endometriosis in several sections (see Figure 6A). As the method detects early-produced free radicals, the method performs effectively in the detection of early or mild endometriosis. In this cohort, 9 of the 10 patients had early or mild disease. Taking into account that there are no differences in the range between 500 and 600 nm, the maximum in the ranges 625-635, 825-835 and 872-878 increase altering the shape of the spectrum compared to the control. Within the second range between 953-1000, wherein there is a statistically significant increase p-value Wilcoxon <0.0001. This indicates that the major radical species modifying haemoglobin during endometriosis is O₂^{●-,} ,with no change in absorbance consistent with the significant presence of others. Moreover, the haemoglobin concentration decreases in the erythrocytes from women with endometriosis (Figure 6B).

Consequently, the measurement of the area under the curve is affected showing a significant increase (Figure 6B). The detection capability of the method in a cohort of 10 patients is relatively good with an ROC curve with an area of 0.85.

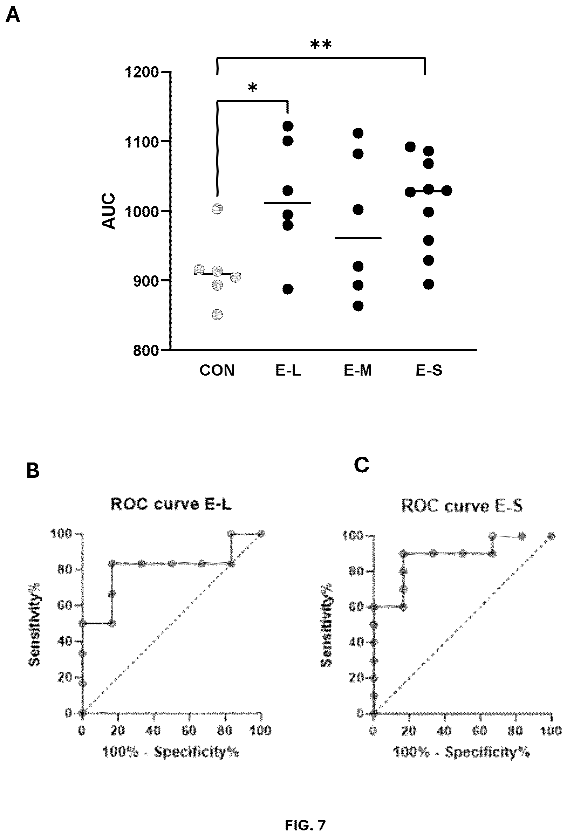

Consistent with the ability to detect early free radicals, this method discriminates disease stages. In a second cohort (CON=5, ENDO=22) the ROC curve obtained an area of 0.80. Analogous to the previous cohort the ability of the method to detect mild or early disease is good (Figure 7A and Figure 7B), the ROC curve shows an area of 0.80 for detecting mild cases. This ability is not matched by other systems for detection of endometriosis in circulating blood or by detection of free radicals that have been tested in patients with severe involvement in which surgical resection is often indicated.

### CONCLUSION

The absorbance of the haemoglobin spectrum of the blood of women with endometriosis in the NIR between 500 and 1000 nm is different from the spectrum of women without the disease and this allows differentiation between women with and without the disease.

## Claims

1. A method for identifying free radicals present in a pathological process suffered by a subject comprising:
(a) determining the absorption spectrum of haemoglobin (Hb) between 500 and 1 000 nm in a sample of venous blood isolated from the subject, and
(b) comparing the absorption spectrum obtained in step (a) with the absorption spectrum of Hb between 500 and 1 000 nm obtained in a venous blood sample from a population not suffering from the pathological process,
wherein:
- If the absorption spectrum of Hb determined in step (a) shows a significant decrease in absorbance at 955, 985, and 1000 nm, resulting in a decrease in the area under the curve between 955 and 1000 nm, then the major free radical is hydrogen peroxide (H₂O₂),.
- If the absorption spectrum of Hb determined in step (a) shows a significant increase in absorbance at 640, 655, 670, 685, 700 and 715 nm, resulting in an increase in the area under the curve between 640 and 715 nm, then the major free radical is peroxynitrite (-ONOO-),
- If the absorption spectrum of Hb determined in step (a) shows a significant increase in absorbance at 625, 640, and 655 nm, resulting in an increase in the area under the curve between 625 and 655 nm, then the major free radical is superoxide (O₂^{●-}),
- If the absorption spectrum of Hb determined in step (a) shows a significant decrease over the spectrum between 600 and 1000 nm, resulting in a decrease in the area under the curve between 600 and 1000 nm, then the major free radical is the nitric oxide (NO^{●-}).

2. The method according to claim 1, wherein the pathological process is endometriosis, cancer, an inflammatory lung disease, an inflammatory kidney disease, or an inflammatory skin disease.

3. *In vitro* method according to claim 1 or 2, wherein the subject is a mammal, in particular a primate or a canid, more particularly a human being.

4. Use of the absorption spectrum of haemoglobin between 500 and 1000 nm for the *in vitro* identification of free radicals present in a pathological process suffered by a subject.

5. Use according to claim 4, wherein the absorption spectrum of haemoglobin between 500 and 1000 nm in the presence of free radicals comprises the following changes:
- If the absorption spectrum of Hb determined in step (a) shows a significant decrease in absorbance at 955, 985, and 1000 nm, resulting in a decrease in the area under the curve between 955 and 1000 nm, then the major free radical is hydrogen peroxide (H₂O₂.),
- If the absorption spectrum of Hb determined in step (a) shows a significant increase in absorbance at 640, 655, 670, 685, 700 and 715 nm, resulting in an increase in the area under the curve between 640 and 715 nm, then the major free radical is peroxynitrite (-ONOO-),
- If the absorption spectrum of Hb determined in step (a) shows a significant increase in absorbance at 625, 640, and 655 nm, resulting in an increase in the area under the curve between 625 and 655 nm, then the major free radical is superoxide (O₂^{●-}),
- If the absorption spectrum of Hb determined in step (a) shows a significant decrease over the spectrum between 600 and 1000, resulting in a decrease in the area under the curve between 600 and 1000 nm, then the major free radical is the nitric oxide (NO^{●}).

6. Use according to claim 4 or 5, wherein the pathological process is endometriosis, cancer, an inflammatory lung disease, an inflammatory kidney disease, or an inflammatory skin disease.

7. Use according to any one of claims 4 to 6, wherein the subject is a mammal, in particular a primate or a canid, more particularly a human being.

8. An *in vitro* method for diagnosing the pathology suffered by a subject comprising
(a) determining the pattern of free radicals present in a venous blood sample isolated from a subject suffering from the pathology to be diagnosed by the method of any one of claims 1 to 3,
(b) determining the pattern of free radicals present in a venous blood sample isolated from a subject to be diagnosed by the method of any one of claims 1 to 3, and
(c) compare the pattern of free radicals obtained in step (a) with the pattern of free radicals present in the subject to be diagnosed obtained in step (b),
wherein if the subject to be diagnosed has the same pattern of free radicals as the subject with the pathology, then the subject is diagnosed with the pathology.

9. *In vitro* method according to claim 8, wherein the pathology to be diagnosed is endometriosis, cancer, an inflammatory lung disease, an inflammatory kidney disease, or an inflammatory skin disease.

10. The in vitro method according to claim 9, wherein if the pathology is endometriosis, then:
- the area under the absorbance curve between 625 and 655 nm for haemoglobin in NIR is greater than the area under the curve obtained from a non-endometriosis subject, which is indicative of increased O₂^{●-} , and
- the area under the absorbance curve between 900 and 1000 nm for haemoglobin in NIR is smaller than the area under the curve obtained from a non-endometriosis subject, which is indicative of a decrease in NO.

11. Method according to any one of claims 7 to 10, wherein the subject is a mammal, in particular a primate or a canid, more particularly a human being.

12. Use of the absorption spectrum of haemoglobin between 500 and 1000 nm for *in vitro* diagnosis of a pathology in a subject.

13. Use according to claim 12, wherein the absorption spectrum of haemoglobin between 500 and 1000 nm in the presence of free radicals comprises the following changes:
- If the absorption spectrum of Hb determined in step (a) shows a significant decrease in absorbance at 955, 985, and 1000 nm, resulting in a decrease in the area under the curve between 955 and 1000 nm, then the major free radical is hydrogen peroxide (H₂O₂),.
- If the absorption spectrum of Hb determined in step (a) shows a significant increase in absorbance at 640, 655, 670, 685, 700 and 715 nm, resulting in an increase in the area under the curve between 640 and 715 nm, then the major free radical is peroxynitrite (-ONOO-),
- If the absorption spectrum of Hb determined in step (a) shows a significant increase in absorbance at 625, 640, and 655 nm, resulting in an increase in the area under the curve between 625 and 655 nm, then the major free radical is superoxide (O₂^{●}),
- If the absorption spectrum of Hb determined in step (a) shows a significant decrease over the spectrum between 600 and 1000, resulting in a decrease in the area under the curve between 600 and 1000 nm, then the major free radical is the nitric oxide (NO^{●-}).

14. Use according to claim 12 or 13, wherein the subject is a mammal, in particular a primate or a canid, more particularly a human being.

15. Use according to any one of claims 12 to 14, wherein the pathology is endometriosis, cancer, an inflammatory lung disease, an inflammatory kidney disease, or an inflammatory skin disease.

16. Use according to claim 15, wherein if the pathology is endometriosis, then:
- the area under the absorbance curve between 625 and 655 nm for haemoglobin in the NIR is greater than the area under the curve obtained from a non-endometriosis subject, which is indicative of increased O₂^{●-}, and
- the area under the absorbance curve between 900 and 1.000 nm for haemoglobin in NIR is smaller than the area under the curve obtained from a non-endometriosis subject, which is indicative of a decrease in NO.
